# EUROPEAN PATENT SPECIFICATION

(11) **EP 4 137 047 B1**
(45) Date of publication and mention of the grant of the patent: **30.07.2025**
(21) Application number: 21191816.4
(22) Date of filing: 18.08.2021
(51) Int. Cl.: A61B 5/145, A61B 5/00

(54) **INSERTION DEVICE FOR TRANSCUTANEOUS INSERTION**
EINFÜHRVORRICHTUNG ZUR TRANSKUTANEN EINFÜHRUNG
DISPOSITIF D'INSERTION POUR INSERTION TRANSCUTANÉE

(43) Date of publication of application: 22.02.2023
(73) Proprietor: Roche Diabetes Care GmbH, 68305 Mannheim (DE); F. Hoffmann-La Roche AG, 4070 Basel (CH)
(72) Inventor: Roscher, Olaf, 68305 Mannheim (DE)
(74) Representative: Grünecker Patent- und Rechtsanwälte PartG mbB

(56) References cited:
- US-A1- 2010 179 409
- US-A1- 2011 028 816
- US-A1- 2018 353 684
- US-A1- 2021 187 189

## Description

The invention relates to a device for transcutaneous insertion. Such devices for insertion may be positioned in a subcutaneous position, and e.g. be used to measure physical parameters, e.g. the glucose value, of a user and/or be positioned in a transcutaneous position, e.g. for transcutaneous delivery of a drug, e.g. insulin. Typically, such devices comprise a flexible insertion piece for transcutaneous insertion, which is configured to remain in a transcutaneous (e.g. subcutaneous) position during use, typically in a particular depth of the skin.

US 2010/179409 A1 discloses systems and methods for replacing signal artifacts in a glucose sensor data stream. US 2021/187189 A1 discloses a connected drug delivery system for erythropoietin stimulating agents.

The flexible insertion piece may be inserted into the skin with the help of a placement assembly. For example a placement assembly may comprise a hollow insertion needle, which may have an open side to allow easy retraction. The hollow insertion needle may envelop the flexible insertion piece during the transcutaneous insertion process. After completion of the insertion, the placement assembly comprising the hollow insertion needle, or a part thereof such as the hollow insertion needle, may then be retracted such that the flexible insertion piece, in particular only the flexible insertion piece, remains inserted in the skin. Another example for a placement assembly may comprise an insertion needle or other tool, preferably having a pointed tip, which may be inserted into the flexible insertion piece during the transcutaneous insertion process. The insertion needle or other tool may not be hollow. After completion of the insertion, the placement assembly or part thereof, such as the insertion needle or tool, may be retracted such that the flexible insertion piece, in particular only the flexible insertion piece, remains inserted in the skin. The flexible insertion piece, in particular the parts of it remaining outside of the skin, may then be fixed to the skin, e.g. by a patch.

However, when inserting the insertion piece into the skin, the user may not have a possibility to check the positioning of the insertion piece in the skin. In some cases, the insertion may lead to an improper placement in the skin, resulting in improper functioning. In particular, for a flexible insertion piece used for measuring, this may lead to erroneous measurement results. In the event the flexible insertion piece is a drug delivery cannula, erroneous placement into the skin may result in partial or complete occlusion of the cannula so that there is a risk that delivery results in wrong amounts of delivered drug.

Such an improper placement in the skin may be detected using secondary means, e.g. carrying out a control measurement in an independent manner in order to check the result

(e.g. carrying out a glucose level measurement by drawing blood or measuring drug levels after insertion of the drug). However, this leads to the need of additional devices for this check and is tedious for the user. Additionally, such an improper placement may also happen after even a correct insertion of the flexible insertion piece and after a check has been carried out, e.g. due to the flexible insertion piece moving when the skin moves, in particular when the skin moves such that the insertion piece's position in the skin depth changes and/or the axial position of the attached device. In such cases, the deformation of the flexible insertion piece may not be detected and lead to erroneous results, which may harm the user's health.

Such improper functioning may be caused or indicated by a deformation of the flexible insertion piece.

Thus, there is a need for improved means and methods for determining proper insertion of a flexible insertion piece during and after its insertion into the skin that avoids the drawbacks of the prior art. This problem is solved by the means and methods provided by the present invention as specified in the claims and herein below.

Hence, the invention comprises a device for transcutaneous insertion, in particular e.g. a device for transcutaneous delivery of a drug or for transcutaneous measurement of a physical parameter such as an analyte concentration, comprising a flexible insertion piece for transcutaneous insertion (also called insertion piece in the following), which comprises a deformation measurement device for detecting one or more deformation signals. In particular, the one or more deformation signals that may allow determining whether a deformation is present in the deformation measurement device, and thus in at least a part of the insertion piece.

In particular, the deformation measurement device may be triggered, e.g. by one or more start signals, e.g. application of a voltage or current, or may be connected to a start signal, e.g. a voltage, continuously. From the deformation measurement device, one or more deformation signals may be received in response to the triggering. These one or more deformation signals may e.g. comprise a voltage, a current or a resistance.

From the one or more deformation signals, a deformation value may then be determined. The deformation value may in particular correspond to a value measured from the deformation signals, e.g. a measured voltage, current or resistance, or may be derived therefrom, e.g. be calculated using information from the one or more deformation signals.

Additional information, e.g. information about the deformation measurement device and/or the triggering, e.g. the one or more start signals, may additionally be used in the calculation of a deformation value derived from the one or more deformation signals. The derived deformation value may thus e.g. describe an amount of the deformation. The deformation value may be one-dimensional (correspond to one value) or have more than one dimension (e.g. correspond to two one-dimensional or more than two one-dimensional values).

The determined deformation value may be compared with a reference value, which may be a predetermined reference value.

If the determined deformation value fulfills a predetermined condition with regard to the reference value, a deformation may be present, and an indication may be provided to a user or a system that the flexible insertion piece has been improperly positioned. In particular, if the determined deformation value fulfills a predetermined condition with regard to the reference value, at least one of the following steps may be conducted: an alarm may be issued which is detectable on the device for transcutaneous insertion; an alarm may be issued which is transmitted to a display device and the device for transcutaneous insertion may be deactivated. If the deformation value does not fulfill a predetermined condition with regard to the reference value, a deformation may not be present and no notification may be given, or an ok-notification may be given. An alarm which is detectable on the device for transcutaneous insertion may in particular comprise a vibration and/or a light, e.g. a flashing light or a switched on light, e.g. a red light, and/or an indication on a display.

Such a deformation measurement device comprised by the device for transcutaneous insertion may for example be mounted on or in the flexible insertion piece.

In this text, the predetermined condition that the determined deformation may fulfill with regard to the reference value may comprise or be that the determined deformation value is higher than the reference value or that the absolute value of the difference between the determined reference value and the reference value corresponds to or exceeds an, optionally predetermined, threshold. This threshold may be 0 or greater than 0. The predetermined condition may also comprise or be that the determined deformation value is above a first reference value or below a second reference value, or that the determined deformation value is within a certain reference interval.

When a user notes an issued alarm or is provided with an indication that the flexible insertion piece has been improperly positioned, the user may then take further steps to avoid malfunctioning of the device, e.g. re-position the flexible insertion piece by removing it and positioning it in a different place or positioning a different flexible insertion piece in another part of the skin.

In this text, the term "transcutaneous" may describe a passing or entering through the skin, i.e. an insertion of the flexible insertion piece into the skin, e.g. for measurement of a physical parameter or delivery of a drug under the skin (subcutaneous). The term "deformation" may refer to mechanical deformation like bending, resulting in mechanical stress of the material.

A flexible insertion piece may in particular be made of a plastic or synthetic material, and may allow a bending of the flexible insertion piece to an angle of at least 45°, for example to at least 90°, in particular more than 90° over its length, while inserted into skin and without tearing the skin. The length of the flexible insertion piece may, for example, be between 1 mm and 35 mm, e.g. between 8 mm and 20 mm, in particular between 8 mm and 16 mm. The flexible insertion piece may in particular have a low bending stiffness allowing a bending of the flexible insertion piece to an angle of about 90°, in particular between 75° and 135°, in particular between 80° and 100° using an, in particular lateral, force of between 0.001 N and 1 N, e.g. of between 0.005 N and 0.5 N, in particular of between 0.01 N and 0.1 N.

The flexible insertion piece may be hollow, e.g. comprise a soft cannula for drug insertion, e.g. insulin, or may be solid, e.g. in the form of a flexible insertion piece comprising an analyte sensor. A soft cannula may, in particular, be made of or comprise Teflon or silicone. A flexible insertion piece comprising an analyte sensor may, in particular, be made of or comprise one or several foil or film layers made up of or comprising polyester, e.g. polyethylene terephthalate (PET), in particular biaxially oriented polyethylene terephthalate (PET), e.g. a biaxially oriented polyethylene terephthalate film, or other plastic or insulating materials. For example, a flexible insertion piece may be made of or comprise Melinex films (Melinex is a registered trademark of DuPont Teijin Films).

The device for transcutaneous insertion may be a medical device. It may comprise a patch for or other fixing aid, e.g. a layer of adhesive, for affixing a part of the flexible insertion piece remaining outside of the skin to the skin. Additionally or alternatively, the device for transcutaneous insertion may comprise a placement assembly as described above. The device for transcutaneous insertion may optionally comprise a housing and/or a drug infusion pump, in particular a drug infusion pump in a housing. The housing may optionally be configured to cover at least part of a patch or other fixing aid when the device for transcutaneous insertion is inserted into a user's skin.

A deformation measurement device may, for example, comprise (micro-)conductors with predetermined breaking points, which break if a bending of the flexible insertion piece exceeds a certain threshold bending.

A deformation measurement device may alternatively or additionally comprise two or more markings on the flexible insertion piece, the position of which may be visually checked (e.g. by the user himself or using a camera). If the distance of the positions deviates from a known distance of the marks (reference value) more than a certain threshold, this indicates an adverse deformation of the flexible insertion piece.

Such a deformation measurement device according to the invention may in particular comprise a strain measurement device, e.g. for detecting a bending of the insertion piece. Strain measurement devices as such are known and for example available from HBM Deutschland, Hottinger Brüel & Kjaer GmbH, Darmstadt, Germany. Strain measurement devices of the invention may for example correspond to strain measurement devices used in strain gauges. A strain measurement device of the invention may e.g. comprise a meander-shaped pattern of a conductor on an insulating layer or some other arrangement of a conductor in a particular direction on an insulating layer.

This meander-shaped pattern or other arrangement of a conductor may, in the following, also be referred to as "measurement grid".

A meander-shaped pattern of a conductor of the invention may be arranged along a certain direction. When stretching the layer comprising the conductor into that direction, the resistance of the conductor increases, while on the other hand, when compressing the layer comprising the conductor into that direction, the resistance of the conductor decreases. A deformation value may hence be the resistance of the strain measurement device.

Particular patterns, which may be used for strain gauges can, with proper size adjusting (i.e. typically miniaturization), be mounted on or in a flexible insertion piece according to the invention. Other patterns which may be used for the present invention are any patterns having repeated parallel lines of a continuous conductor arranged parallel to each other with the same height, or at least approximately parallel to each other (deviation from parallel e.g. not more than 10°, or not more than 20°). Such arrangements may, in particular, allow recognizing a change of resistance when the conductor is deformed.

While in the following, meander-shaped patterns are discussed as a particular example for strain measurement devices of the invention, it is understood that other patterns serving the same purpose mentioned above, and also for example a more complex combination of more than one meander-shaped or similar pattern, e.g. as used in a membrane rosette strain gauge, may be used in the present invention.

Typically, the strain measurement device of the invention is positioned in the area of the flexible insertion piece in which a deformation would be of particular interest or harmful to the functioning of the device. In particular, this typically comprises or consists of the region of the flexible insertion piece inserted into the skin and the region adjacent to it, which is not supposed to be inserted. The strain measurement device of the invention may alternatively or additionally be positioned in the region of the flexible insertion piece which is intended to bend during insertion or which is bent before and/or after insertion of the flexible insertion piece.

Typically, such a flexible insertion piece for transcutaneous insertion comprises an insertion area configured to extend from a tip inserted into the skin to the surface of the skin, a connection area and an attachment area. The tip is the part of the flexible insertion piece which is intended to be inserted into the skin deepest in use. It may comprise an, optionally electronic, analyte measurement arrangement of an analyte sensor and/or an opening, e.g. the opening may be used or usable for administering a drug into the skin. The device for transcutaneous insertion may be configured such that, when inserted into the skin, the tip is placed subcutaneously.

The attachment area is arranged on the opposite end of the tip of the flexible insertion piece. Optionally, the attachment area is configured to be attached to electronics and/or a drug reservoir, e.g. the electronics allowing sending to and receiving signals from the flexible insertion piece, in particular the deformation measurement device and/or the drug reservoir allowing delivering of a drug. The attachment area may comprise contact regions for electrical connections, e.g. for connecting it to contacts within a housing or a patch, and/or one or more connection regions or points for connecting to a drug reservoir.

The connection area connects the insertion area with the attachment area. It may, in particular, be configured to be bent to allow attaching the flexible insertion piece, in particular in the attachment area and/or connection area, along the surface of the user's skin. Typically, the regions of interest for which deformation, in particular a deformation value, is of most interest are the insertion area and/or the connection area. Typically, therefore, the deformation measurement device is arranged such as to allow detecting deformation signals of the insertion area and/or the connection area. In particular, the deformation measurement device may be mounted on or in the insertion area and/or the connection area.

Once the flexible insertion piece has been inserted, the tip usually is not bent as it is already inside the skin. However, movement of the skin or external force applied to the skin and/or to the device for transcutaneous insertion may lead to deformation of the insertion area and/or the connection area of the flexible insertion piece. The attachment area may be less likely to be affected by the force though it is possible that it is deformed as well as, when it is in use, it is usually attached to the user's skin such that this area may not be deformed easily.

In particular, the deformation measurement device of the invention may be arranged such that it allows detecting one or more deformation signals in at least half of the flexible insertion piece, half of the flexible insertion piece or less than half of the flexible insertion piece, in particular in the half or less than half of the flexible insertion piece configured for transcutaneous insertion and immediately adjacent to the part of the flexible insertion piece configured for transcutaneous insertion, in particular in the insertion and/or connection area.

The signals received from the strain measurement device of the invention may represent electrical resistance which can for example be measured using a Wheatstone Bridge or any other resistance measurement device. The resistance measurement device, in particular a Wheatstone Bridge, may be located in the insertion device for transcutaneous insertion, e.g. integrated into a patch which attaches the flexible insertion piece to the user's skin, or integrated into other electronics , e.g. in a housing or drug infusion pump.

According to the invention, such a strain measurement device, e.g. in a pattern as is known to be used on a strain gauge, can, in particular after proper size adjusting, i.e. typically a miniaturization, be mounted in or on a flexible insertion piece, in particular on an insulating layer of the flexible insertion piece and/or below a protective, e.g. biocompatibility, layer, e.g. in the case of the flexible insertion piece comprising an analyte sensor or a flexible cannula for drug delivery.

Such a strain measurement device may in particular be mounted, optionally, on an insulating layer, in or on the flexible insertion piece using a stereolithographic or printing process.

Such a strain measurement device of the invention may in particular allow detecting a bending of the insertion piece as such a bending of the insertion piece typically leads to a strain (stretching or compression) thereof in the bending area. Hence, a bending of the device (and an approximate amount thereof) can be determined with the strain measurement device of the invention.

In particular, such a strain measurement device may revert to its original properties upon bending and unbending it. In particular, after bending and unbending it once, it may not be damaged, but rather still be usable. It may in particular be possible to detect a deformation (bending), and detect a change back to the original (un-deformed/unbent) state and/or a change to an even more deformed (bent) state in subsequent measurements.

The strain measurement device may be suitable for continuous measurement, e.g. measurement in certain time intervals during transcutaneous insertion of the flexible insertion piece, in order to recognize changes in the strain, and in particular hence deformation, and through it changes in the positioning of the flexible insertion piece. This may allow deactivating the device for transcutaneous insertion and/or transmitting a notification, e.g. issuing an alarm or message, to the user or the system, e.g. by transmitting a notification to a display or sending another message, e.g. cautioning the user or system that the positioning of the flexible insertion piece may be improper and that it should be checked.

The strain measurement device may in particular be an electronic strain measurement device, which can optionally be attachable to an electronic device (electronics) outside of the flexible insertion piece, e.g. in a patch, in a housing, in particular of an analyte sensor device or in a drug infusion pump.

According to the invention, the deformation measurement device comprises two strain measurement devices The deformation measurement device may also comprise three or more strain measurement devices.

The two, three or more strain measurement devices may be wired each individually, i.e. may be controlled and/or may be monitored separate from each other, or one, two, three or more of them may be wired individually. Alternatively or additionally, two, three, more, or all of the strain measurement devices may be connected in parallel and/or two, three, more or all of the strain measurement devices may be connected in series.

Connection in series and/or in parallel is associated with the advantage that the number of wires can be reduced to two. However, while such arrangement in series allows determination of a deformation in at least one of the strain measurement devices, it is not possible to identify the particular strain measurement device where the deformation signal stems from. In contrast, individual wiring is associated with the advantage that it allows for identification of the particular strain measurement device where the deformation signal stems from. On the other hand, the number of wires used to connect all strain measurement devices is increased in comparison to the serial connection arrangement.

The flexible insertion piece of the invention may, in particular in an unbent state, have a longitudinal axis. In a bent state of the flexible insertion piece, the longitudinal axis may be bent as well. This longitudinal axis typically corresponds to the direction along which the flexible insertion piece is to be introduced into the skin. When viewed in a cross section perpendicular to its longitudinal axis, the flexible insertion piece may have one of the following different shapes: a line or very thin rectangle, which corresponds to a flat or strip-formed configuration of the flexible insertion piece, a circular cross section which corresponds to a cylindrical configuration which is also named wire configuration of the flexible insertion piece, an oval shape and a regular or irregular polygonal shape with at least 3 corners which corresponds to a prism shape of the flexible insertion piece.

For a flat flexible insertion piece of the invention, e.g. an insertion piece made up from at least 2 layers in a flat configuration, a strain measurement device may be mounted on a first flat side and another strain measurement device may be mounted on the opposite flat side. For example, when detecting deformation signals, in particular receiving one or more signals, of both of those strain measurement devices, a bending of the flexible insertion piece along the flat side may be recognized by the deformation value determined from the one or more signal(s) of the first strain measurement device indicating a stretching, e.g. an increase in resistance with regard to a reference value, and the deformation value determined from the one or more signal(s) of the second strain measurement device indicating a compression, e.g. a decrease in resistance with regard to a reference value. Hence, a bending of the flexible insertion piece may be distinguished from an overall stretching thereof.

On a flexible insertion piece having a longitudinal axis, two, three or more strain measurement devices may be arranged at different angles around the longitudinal axis of the flexible insertion piece. For example in a flat (strip-formed) flexible insertion piece, they may be arranged under an angle of about 180°, meaning of between 90° and 270°, e.g. between 135° and 225°, e.g. on opposed sides of the flat insertion piece. In another example, in a wire configured flexible insertion piece, they may be arranged under an angle of about 90° from each other, i.e. between 45° and 135°, in particular between 60 and 120°, for example between 75° and 105°. The two, three, or more strain measurement devices, or some of them, may in particular be arranged in parallel.

A parallel arrangement may in particular mean that they are arranged on the same height with regard to the longitudinal axis of the flexible insertion device. Such a parallel arrangement of two, three or more strain measurement devices may in particular allow a monitoring of a deformation in two, three or more directions, distinct from each other. For example, for a wire configured flexible insertion piece having strain measurement devices arranged under an angle of 90° from each other, deformation under an angle of 90 ° from each other may be monitored. Angles in between, e.g. deformation at an angle between the two strain measurement devices may also be recognized, by both of them indicating some strain in particular if the angle between them deviates from 180°, is e.g. about 90°. Such an arrangement may thus in particular allow recognizing any bending of the longitudinal axis of the insertion piece at a height where the deformation measurement devices are arranged.

On a flexible insertion piece having a longitudinal axis, the axis of the deformation measurement device, in particular a strain measurement device, may be oriented along a longitudinal axis of the flexible insertion piece. This may allow a recognition of a deformation (bending) of the longitudinal axis. This may in particular be of relevance as this is the axis along which deformation (bending) is most likely and most likely to affect the proper positioning of the flexible insertion piece in the skin.

An arrangement of a deformation measurement device, in particular a strain measurement device, along an axis may mean that it is arranged such as to allow detecting a deformation of said axis, i.e. when said axis is deformed. In the example of a strain measurement device comprising a meander-shaped conductor, an arrangement of said strain measurement device along an axis means that the long parts of the meander pattern are arranged along, in particular parallel to, said axis.

On a flexible insertion piece having a longitudinal axis, the wiring attached to the deformation measurement device may be oriented along a longitudinal axis of the flexible insertion piece. This may have the advantage that the wiring can be orderly arranged along the longitudinal axis and/or arranged parallel to any other wiring also necessary on the flexible insertion piece, e.g. for other measurements. In particular, the wiring for the deformation measurement device may be arranged in the same layer as other wiring on the flexible insertion piece.

Optionally, the deformation measurement device is arranged on the outside of the flexible insertion piece or on the inside of the flexible insertion piece off-centered, i.e. not in the center of the flexible insertion piece, in particular away from the middle layer, e.g. of a flat configuration. This may in particular mean that it is arranged away from the longitudinal axis, in particular on the outermost layer having conductive wiring (a protective layer, e.g. a covering insulating layer and/or biocompatibility layer may or may not be present), or mounted on one of the two, three or four outermost insulating layers. Outermost in this regard may in particular mean closest to the user's skin (in a use position) and/or away from the flexible insertion piece's center.

The device for transcutaneous insertion may in particular be an analyte measurement sensor device such as a continuous analyte measurement sensor device. The continuous analyte measurement sensor device may comprise a transcutaneous analyte sensor, in particular may comprise an electronic analyte measurement arrangement, e.g. arranged on the tip.

The analyte may be glucose, a ketone, a cholesterol, a triglyceride, and lactate, particularly preferred is glucose. Thus, for example, the continuous analyte measurement sensor (CAM sensor) may in particular be a continuous glucose measurement sensor (CGM sensor). The CAM sensor may comprise an electronic analyte measurement arrangement, wherein the electronic analyte measurement arrangement may, for example, comprise two or more electrodes. The CAM sensor may in particular be configured for subcutaneous continuous analyte measurement (subcutaneous insertion). The part of the CAM sensor comprising the electronic analyte measurement arrangement, e.g. two or more electrodes, is typically configured for subcutaneous insertion. The CAM sensor may further comprise wiring for addressing of the electronic analyte measurement arrangement and the deformation measurement device. The wiring is typically also present in the connection area and optionally the attachment area. Typically, the wiring is arranged to be connected to electronics, e.g. present in a patch attaching the CAM sensor to the skin.

For example, the electronic analyte measurement arrangement and the deformation measurement device may be mounted on a flexible carrier, which may optionally comprise several foil or film layers (which may in particular be insulating layers). The flexible insertion piece may comprise the flexible carrier.

The analyte measurement arrangement and the deformation measurement device may be arranged on the carrier separate from each other. In particular, they may be mounted on separate layers of the foil or film, or on the same layer in separate areas. The wiring of the analyte measurement arrangement and the deformation measurement device may be arranged in one layer, which is different from the layer on which the glucose measurement arrangement is mounted and/or which is different from the layer on which the deformation measurement device is mounted. Optionally, the deformation measurement device is further outside than the analyte measurement arrangement, in particular configured to be in use closer to the user's skin and further away from the longitudinal axis, which may also be called neutral axis, which is not stretched, or the middle of the carrier in a direction perpendicular to the direction of insertion.

The flexible insertion piece comprised by the device for transcutaneous insertion may be or comprise a flexible cannula, e.g. a Teflon or silicone cannula, used for transcutaneous, delivery of a drug. It may have a circular or oval cross section, which may in particular be measured perpendicular to the longitudinal axis or direction of insertion thereof. It may, in particular, be a flexible cannula of a drug infusion pump or in particular of a body-worn drug infusion patch pump, for example for the infusion of insulin. One end of the flexible cannula may be attachable or attached to a drug infusion pump, a patch or in another manner outside of the skin while the other part of the cannula may be configured to remain inside the skin of a user, in particular subcutaneously. The deformation measurement device may be mounted on the outside of the cannula, i.e. the wall of the cannula, which - during use - is to be turned towards the skin of the user, and optionally not in contact with the drug delivered through the cannula. The deformation measurement device may be covered by a protective, e.g. biocompatibility, layer on the outside of the cannula, such that - during use - the deformation measurement device is not in direct contact with the user's skin or other bodily fluids.

The invention further comprises a transcutaneous insertion system comprising a device for transcutaneous insertion as described above, an optional display device and a memory. The memory comprises instructions that, when executed, cause one or more processors to receive one or more signals from the deformation measurement device, determine a deformation value of the flexible insertion piece from the one or more signals and optionally transmit a notification to the display device.

The memory may further comprise instructions that, when executed, cause one or more processors to compare the determined deformation value with a reference value. If the determined deformation value fulfills a predetermined condition with regard to the reference value, an indication may be provided to a user or a system that the flexible insertion piece has been improperly positioned. In particular, if the determined deformation value fulfills a predetermined condition with regard to the reference value, at least one of the following steps may be conducted: an alarm may be issued which is detectable on the device for transcutaneous insertion; an alarm may be issued which is transmitted to a display device and the device for transcutaneous insertion may be deactivated.

The signals from the deformation measurement device may, for example, be received in response to measurement signals, in particular e.g. in form of a triggering, e.g. start signal(s), from electronics comprised in the system and/or controlled by the memory. The deformation value of the flexible insertion piece may e.g. be determined by determination of a measurement value (e.g. resistance of one or more strain measurement devices) and comparison thereof to a reference value (e.g. reference resistance), which may be known from previous calibration by a previous measurement or from a factory calibration. A measurement, e.g. resistance measurement of one or more strain measurement devices, may, for example, be carried out by a Wheatstone Bridge, which may be comprised in the transcutaneous insertion system.

The memory may be comprised in a patch configured to attach a part of the device for transcutaneous insertion to the skin of a user, and/or can be part of an, in particular external, electronic device (electronics) that is attachable or attached to the patch and/or the memory may be comprised in another part. The patch may be configured to transfer data wirelessly to an external device, e.g. a mobile computing device, e.g. cell phone. The memory may alternatively be comprised in an external device, e.g. a cell phone or a remote computer, and/or a part of the memory may be comprised in a patch and part of it may be comprised in an external device.

The device for transcutaneous insertion of the transcutaneous insertion system may in particular be an analyte sensor such as a continuous analyte measurement sensor, e.g. as described above. It may in particular be a continuous glucose measurement sensor (CGM sensor) as described above. The optional display device and the memory may be comprised in a housing adapted for placement adjacent to the skin of a user. The housing may be adapted for receiving at least a portion of the flexible insertion piece, in particular at least a portion of the analyte sensor, e.g. the CAM sensor. The housing may additionally or alternatively be adapted for contacting at least a portion of the analyte, e.g. CAM, sensor. Alternatively or additionally, the display device of the system may correspond with an external display device, e.g. a proprietary device, in particular a locked down phone or device, and/or a display device of a mobile device, e.g. a smartphone or tablet, or a consumer electronic device, e.g. a PC, monitor, TV.

The system may comprise a device for transcutaneous insertion and the memory may comprise instructions that, when executed, cause one or more processors to carry out the steps described with regard to the device for transcutaneous insertion above.

The invention further comprises a method of detecting deformation of at least a part of the insertion piece of a device for transcutaneous insertion, in particular a device for transcutaneous insertion as previously described. The method comprises receiving one or more signals from a deformation measurement device which may be comprised by, in particular e.g. mounted on or in, a flexible insertion piece for insertion; determining a deformation value of the flexible insertion piece from the one or more signals; and optionally transmitting a notification to a display device. Additional steps may be carried out corresponding to the steps as described above for the system.

Further explanations and examples can be found in the drawings, which are not according to scale. Herein,
Fig. 1a shows part of an exemplary flexible insertion piece for transcutaneous insertion
Fig. 1b shows the positioning of an exemplary flexible insertion piece into the skin
Fig 1c shows an exemplary arrangement of a device for transcutaneous insertion
Fig 1d shows part of an exemplary flexible insertion piece for transcutaneous insertion
Fig. 1e shows a cross-section through the exemplary flexible insertion piece of Fig 1d
Figs. 2a, 2b, 2c show exemplary states of a strain measurement device
Fig. 3a shows an exemplary arrangement of a deformation measurement device in a stack of layers
Fig. 3b shows an exemplary flexible insertion piece
Figs. 4a, 4b show exemplary arrangements for connection of two strain measurement devices
Figs 5a, 5b, 5c show exemplary arrangements of strain measurement devices on flexible insertion pieces.
Fig. 6 shows steps which may be comprised in an exemplary method of detecting deformation of at least a part of the insertion piece of an insertion device.

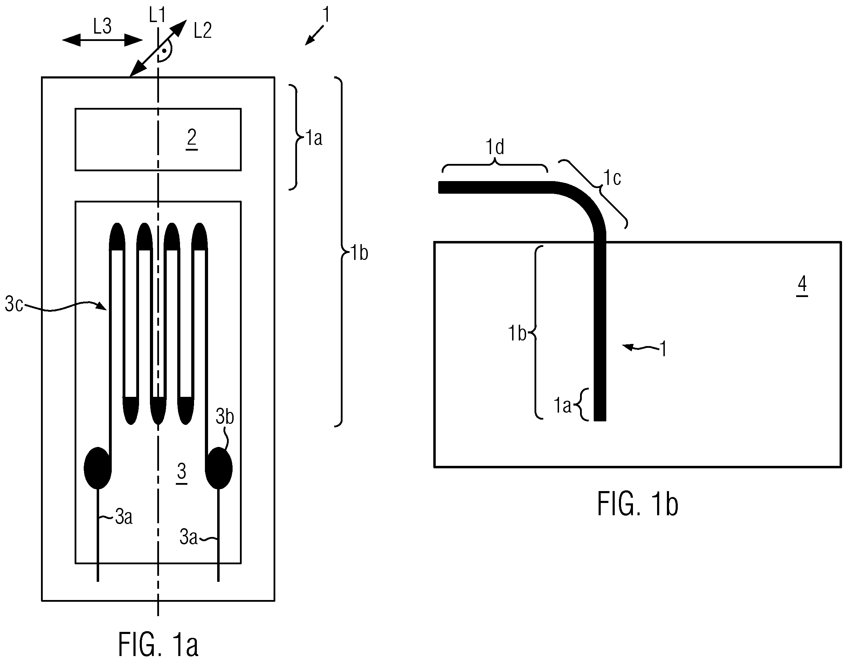

Fig. 1a shows part of an exemplary flexible insertion piece 1. There is a tip 1a in an insertion area 1b configured to run through the skin from the tip towards the outside of the skin. The flexible insertion piece 1 may comprise an analyte sensor such as a continuous analyte measurement (CAM) sensor, e.g. a continuous glucose measurement sensor. The CAM sensor may comprise an electronic analyte measurement arrangement 2, e.g. an electronic glucose measurement arrangement arranged on the flexible insertion piece's tip 1a and/or on the insertion area 1b. The electronic measurement arrangement 2 may in particular be the part of the CAM sensor configured to be subcutaneously placed and exposed to the analyte. In other embodiments, the flexible insertion piece may not comprise an electronic glucose measurement arrangement, but rather a different analyte measurement arrangement or be a cannula (not shown).

A deformation measurement device, here in the form of a strain measurement device 3, is mounted on the flexible insertion piece 1, here exemplarily along at least part of the insertion area 1b, in particular here the insertion area 1b excluding the tip 1a, such that a deformation, in particular a bending, of the deformation area excluding the tip can be determined by the strain measurement device. Alternatively, (not shown) tip 1a and the strain measurement device 3 may overlap each other. The strain measurement device comprises wiring 3a leading towards the connection and attachment area of the insertion piece 1, i.e. towards the direction that is outside the skin during use of the flexible insertion piece. Thus, electronic signals may be sent to and received from the strain measurement device 3.

The insertion piece 1 may, in an unbent state, have a longitudinal axis L1 corresponding to the direction along which the insertion piece is designed to be inserted into the user's skin. Indicated are also axes L2, which is perpendicular to the longitudinal axis L1 and the extension of the meander-shaped pattern, and axis L3 which is perpendicular to L1 and L2, and parallel to the direction into which the meander-shaped pattern extends.

The strain measurement device 3 further comprises optional contact pads 3b connecting the wiring and a meander-shaped pattern of the strain measurement device 3c. In other embodiments, the wiring may be continuous with the meander-shaped pattern of the conductor of the strain measurement device.

The strain measurement device 3 allows identifying deformation information caused by deformation, in particular bending, of the insertion piece in the insertion area in direction of axis L2 or L3.

In particular, for a flat insertion piece 1 having an extension in direction of axis L2 that is distinctly smaller than the extension along axis L3, deformation, in particular bending, is much more likely to occur in direction L2 than in direction L3. Hence, most deformations may also be identified with one strain measurement device. An extension in direction of axis L2 which is distinctly smaller than in direction L2 may in particular mean that the extension of the insertion piece along L2 is less than ½ or less than 1/3 or less than 1/10 than the extension along L3.

Optionally, in order to increase recognition of the deformation information, another strain measurement device may be arranged on the opposite side of insertion piece 1 (not visible), e.g. arranged parallel to the strain measurement device shown. When a deformation occurs now, one deformation measurement device will be stretched while the other will be compressed, resulting in signals indicating an increase and a corresponding decrease of the resistance of the two deformation measurement devices. This may allow a more accurate determination of the deformation.

For insertion pieces 1 having a round cross section along axis L2/L3 (perpendicular to the longitudinal axis), a strain measurement device as shown in Fig. 1a may recognize a deformation along the axis L2, but not along L3. Hence, for such insertion pieces, additional deformation measurement devices, in particular additional strain measurement devices, may be used under different angles around the longitudinal axis L1, e.g. about 90° from each other, for example between 75° and 105° from each other.

In this example, the deformation measurement device 3 is oriented along the longitudinal axis L1 of the flexible insertion piece. Additionally, in this example, the wiring 3a attached to the deformation measurement device 3 is oriented along the longitudinal axis L1 of the flexible insertion piece. In other embodiments (not shown) the deformation measurement device may not be oriented along a longitudinal axis L1 and/or the wiring 3a attached to the deformation measurement device may not be oriented along the longitudinal axis L1.

Fig. 1b shows an example of an insertion piece, e.g. an insertion piece 1 as shown in Fig 1a, inserted into a patient's skin 4. The view corresponds to a view along axis L3 in Fig. 1a, i.e. from the side. The insertion area 1b is (with tip 1a) inserted into the patient's skin 4, while connection area 1c connects the insertion area 1b with attachment area 1d. Attachment area 1d may in particular be configured to be attached to the user's skin and an electronic device which may send to and receive signals from the wiring of the deformation measurement device and optionally any other sensor or measurement arrangement, e.g. a glucose measurement arrangement 2.

In an exemplary arrangement of a CGM sensor, the tip area 1a may have a length of about 0.5 mm, e.g. between 0.4 mm and 0.6 mm, the insertion area including the tip may have a length of about 4.4 mm to about 5.6 mm, the connection area may have a length of about 1 to about 2 mm, and the attachment area 1d may have a length of about 3 to about 8 mm (all lengths measured along the longitudinal axis L1 which, in Fig. 1b, is bent).

In some embodiments, one or more deformation measurement devices may also be present in the connection area in order to identify deformations beyond the required and expected bending (not shown).

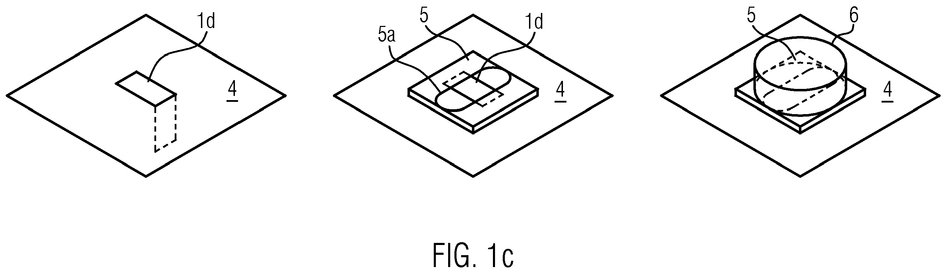

Fig 1c shows an exemplary arrangement of a device for transcutaneous insertion in use, in particular arranged on skin 4 in a use state (inserted into skin 4) in order to explain how such a device for transcutaneous insertion may be used.

In particular, the left image of Fig. 1c shows the flexible insertion piece inserted into skin 4. Although the flexible insertion device may never be inserted into the skin without other parts being present or attached, such other parts of the device for transcutaneous insertion are omitted in the left figure to make it clearer. Visible outside of the skin is the attachment area 1d in full. A part of the connection area may also be visible (not shown). The part of the flexible insertion piece inserted into the skin is indicated with a dashed line.

The middle image of Fig. 1c shows a patch 5 attaching the flexible insertion piece to skin 4, in particular part of the attachment area 1d and optionally part of the connection area (not shown). There may be an optional opening 5a in the patch as indicated. Through such an opening, the flexible insertion piece may be connected to the outside, e.g. contact may be made to the attachment area, in particular to contact regions for electrical connections, e.g. for connecting it to contacts within a housing or a patch, and/or one or more connection regions or points for connecting to a drug reservoir. This may, in particular, allow connecting the flexible insertion piece to an external electronic device (external electronics), in particular an electronic device for sending and receiving signals and/or a drug reservoir.

In the particular example of Fig 1c, the patch attaches the flexible insertion piece to the skin 4 on two sides of the opening. In others, one end of the flexible insertion piece may not be affixed to the skin by the patch, e.g. such that it can extend through the patch (not shown).

Such an opening may not be present in other patches (not shown). If no opening is present, the connection between the flexible insertion piece, in particular the attachment area, and an external electronic device and/or a drug reservoir may be made through the patch, e.g. by the patch connecting to contact regions for electrical connections and/or connection regions for connection to a drug reservoir on the side which is arranged toward the flexible insertion piece in use and having corresponding contact regions for electrical connections and/or connecting regions for connecting to a drug reservoir on the side arranged away from the skin 4 in use, such that the corresponding connections are guided through the patch.

The right image of Fig. 1c shows an additional optional housing 6 arranged on the patch 5 in the use situation. The optional housing may be configured for placement adjacent to skin of a user and optionally for receiving at least a part of an analyte sensor, in particular a CAM sensor. It may optionally comprise electronics and/or memory and an optional display.

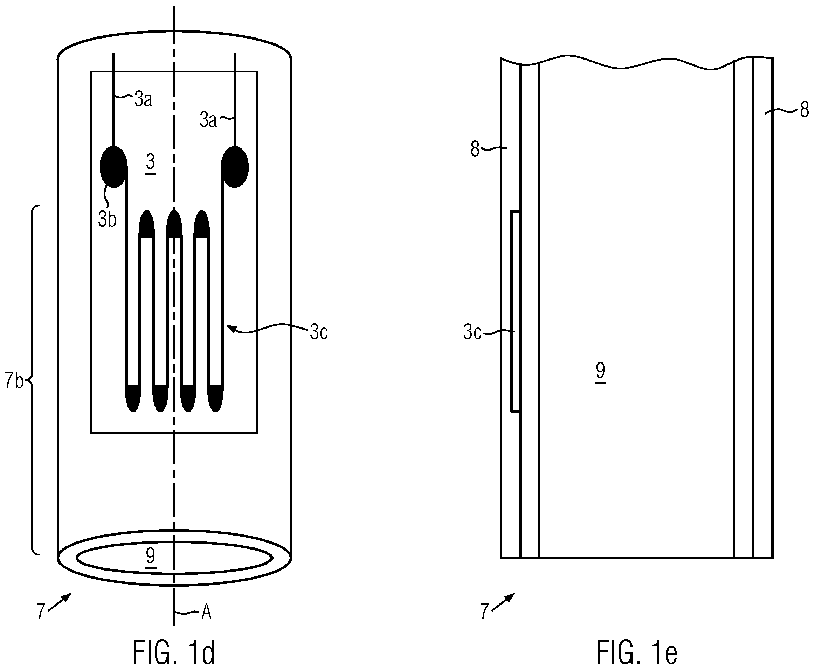

Fig 1d shows part of an exemplary flexible insertion piece for transcutaneous insertion. In the particular example, the flexible insertion piece comprises a flexible cannula 7 for drug delivery, having an opening 9 through which a drug may be delivered.

The insertion area 7b comprises a tip comprising the opening 9, a deformation measurement device 3, exemplarily comprising wiring 3a, pads 3b and a meander-shaped pattern 3c for detecting a deformation signal, in particular for the insertion area 7b.

Fig. 1e shows a cross-section through the exemplary flexible insertion piece of Fig 1d through line A, which is in particular parallel to the longitudinal axis of the flexible insertion piece. Opening 9 inside the cannula may allow delivery of a drug. On the wall of cannula 7, a deformation measurement device 3 is mounted, in the cross section exemplarily indicated through part of its optionally meander-shaped pattern 3c. On top of the cannula's wall is an optional additional protective, e.g. biocompatibility, layer 8. This protective layer 8 may be present around the entire cannula in the insertion region (as indicated in Fig. 1e). It may only cover the deformation measurement device 3 and optionally adjacent parts thereof in other examples.

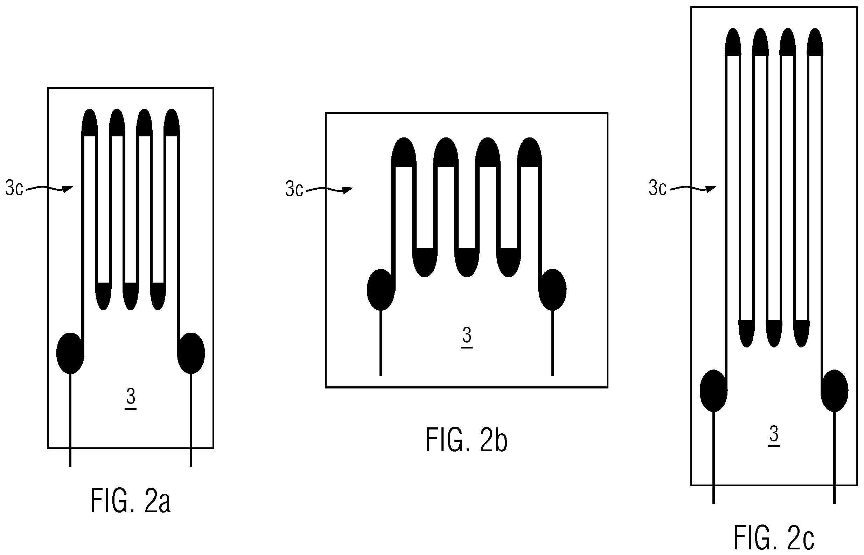

Figs. 2a, 2b and 2c show exemplary states of a deformation measurement device, in particular a strain measurement device in a first, unstrained state (Fig. 2a), a second compressed state (Fig. 2b) and a stretched stated (Fig. 2c). As can be seen, the deformation, in particular strain, leads to a geometrical change of the meander-shaped conductor pattern 3c, hence resulting in a change of the resistance. Compared to the resistance of an undeformed device (Fig. 2a), the deformation of a compressed strain measurement device (Fig. 2b) leads to decreased resistance with regard to the unstretched state (Fig. 1a), whereas the stretched state (Fig. 2c) leads to increased resistance with regard to the unstretched state (Fig. 1a). The resistance of the strain measurement device may be measured using a Wheatstone Bridge or another resistance measurement.

Bending of a flexible insertion device leads to a stretching or a compression of the outer layers thereof. Hence, the resistance after bending may give an indication of the deformation, in particular the bending.

From the received one or more signals from the deformation measurement device, a deformation value, e.g. a measured resistance, may be determined. Comparing the deformation value to a reference value, e.g. a reference resistance, may allow determining whether a deformation is present. In particular, if the deformation value fulfills a predetermined condition with regard to the reference value, e.g. if the absolute value of a difference between the deformation value and the reference value, is larger than a predetermined threshold, this may indicate an adverse deformation, in particular an adverse bending.

The predetermined condition with regard to the reference, in particular a predetermined threshold, may in particular be determined before determining the deformation value. It may be a condition, in particular predetermined threshold, known in advance which may e.g. be looked up in tables or another source. Alternatively or additionally, the predetermined condition, in particular a predetermined threshold, may be determined by a self-learning algorithm. This algorithm may, for example, determine a correlation from past signals of the deformation measurement device and corresponding measured analyte concentrations and/or corresponding control analyte concentrations. The algorithm may determine a predetermined condition, in particular a predetermined threshold, based thereon. The algorithm may take into account additional information, like a user or use situation. This algorithm may be carried out for a particular user, e.g. using just signals of the deformation measurement and/or corresponding measured analyte concentration and/or corresponding control analyte concentrations for a particular user.

Such a determination of the predetermined condition, in particular a predetermined threshold, for a user will lead to a predetermined condition, in particular threshold, adjusted for the particular user, which may in particular thus be adjusted for physiological parameters of the user, e.g. his skin thickness. Due to different skin thicknesses of different users, the flexible insertion piece may have different thresholds indicating that it is still functioning for different users.

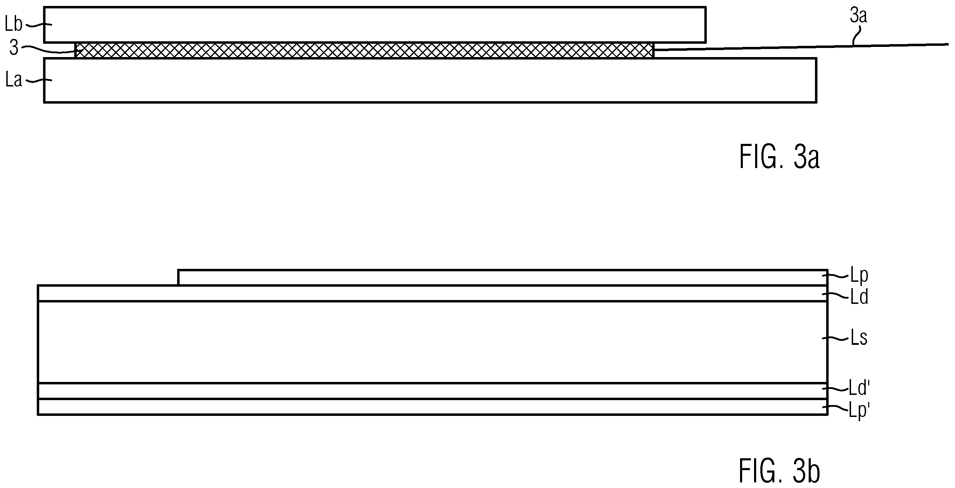

Fig. 3a shows an exemplary arrangement of a deformation measurement device, in particular an exemplary strain measurement device, in a stack of layers. In particular, while the deformation measurement device is optionally arranged on the outside of a flexible insertion piece according to the invention, it may not be the outermost layer.

Rather, the conductor of the strain measurement device 3 may be arranged on an insulating layer La and be covered by an insulating layer Lb such as to protect the conductor against outside influences. Such outside influences may cause a change of the resistance and hence distort the measurement results for the deformation information.

Wiring 3a of the strain measurement device leads toward the surface of the skin allowing a detection of the resistance and comparison thereof with a reference value (deformation information). Insulating layer Lb may be the outermost layer of the flexible insertion piece, whereas insulating layer La may be comprised by the flexible insertion piece or mounted on the flexible insertion piece. Insulating layer Lb may be a protective layer, in particular a biocompatibility layer.

Insulating layer Lb may, in particular, be arranged such as to cover the conductor of the strain measurement device in the entire insertion area (and optionally the tip of the flexible insertion piece). This may reduce influences of the skin on the resistance of the conductor.

Although such an insulating layer Lb on top of the conductor of the strain measurement device is not shown in the other places of this application, in particular the other figures, such a covering insulating layer may, but need not, be present in each and every embodiment disclosed above and below. In particular, there may be an insulating layer Lb on each strain measurement device or there may be an insulating layer Lb on some, but not each, strain measurement device. For example, there may be an insulating layer Lb on the one or each strain measurement device in the insertion section while there may not be an insulating layer Lb on the strain measurement device(s) in the connection area.

Fig. 3b shows an exemplary flexible insertion piece, in particular a cross-section thereof from the side. The flexible insertion piece comprises a stack of (foil or film) layers, in this particular example 5 layers Lp, Ld, Ls, Ld' and Lp'. In other examples, a flexible insertion piece may comprise more or fewer layers.

The flexible insertion piece comprises a substrate layer Ls, which may in particular be arranged centrally, i.e. be arranged such as to contain the flexible insertion piece's longitudinal axis, if it has one. This substrate layer Ls may be stiffer than the outer layers Lp, Ld, Ld', Lp'. It may be an insulating layer. It may optionally comprise (consist of) several independent (e.g. foil or film) layers, wherein one, some or all of them may optionally be insulating layers (not shown). Each of the outer layers Lp, Ld, Ld', Lp' may be made up of one or more separate layers.

In this example, the flexible insertion piece comprises an, in particular insulating, layer Lp working as a protective coating for the deformation measurement device, in particular a strain measurement device. Layer Ld may be or comprise a strain measurement device comprising an insulating layer and the, in particular meander-shaped, conductor of the strain measurement device mounted thereon, wherein the conductor is optionally arranged such as to be in direct contact with the protective coating layer Lp. Alternatively, the, in particular meander-shaped, conductor of the strain measurement device may be the layer Ld, if layer Ls or at least the part of it oriented towards layer Ld is an insulating layer.

In this particular example, layer Lp does not cover the entire layer Ld in order to leave uncovered an optional, in particular continuous, analyte sensor or part thereof, if comprised by the flexible insertion piece, e.g. a glucose measurement arrangement. In other examples, layer Lp may cover the entire layer Ld (not shown).

The flexible insertion piece may further comprise layers Ld' and Lp' on the side of the substrate layer Ls opposite to the side where layer Ld is mounted. In particular, Lp' may be an insulating layer (protective coating) of layer Ld'. Layer Ld' may be or comprise a strain measurement device comprising an insulating layer and the, in particular meander-shaped, conductor of the strain measurement device mounted thereon. The conductor may optionally be arranged such as to be in direct contact with the protective coating layer Lp'. Alternatively, the, in particular meander-shaped, conductor of the strain measurement device may be the layer Ld', if layer Ls or at least the part of it oriented towards layer Ld' is an insulating layer. In other examples, layers Ld' and Lp' may not be present (not shown).

With the particular arrangement shown in Fig. 3b, two strain measurement devices arranged in layer Ld and Ld' are arranged on opposite sides. When the substrate is now deformed, in particular bent in a direction corresponding to up or down in the image, in particular perpendicular to the direction along which the layers are stacked, one strain measurement device will be stretched while the other will be compressed. For example, if bending the flexible insertion piece towards the bottom of the image, the strain measurement device in layer Ld' will be compressed, in particular showing a decreased resistance, while the strain measurement device in layer Ld will be stretched, in particular showing an increased resistance. Presence of two strain measurement devices on opposite sides of a substrate may hence allow an accurate determination of the deformation.

For example, deformation values may be determined from two optionally independent sources, e.g. resistance changes of the two strain measurement devices, and a deformation may only be considered as present if both values are consistent with each other, e.g. if both deformation values indicate the same deformation of the flexible insertion piece. Alternatively or additionally, a deformation value may be determined from comparison of the received signal(s) of the two strain measurement devices with each other.

In one example, the received signals of the two strain measurement devices may be the same in an unbent state. If the difference of received signals of the two strain measurement devices (deformation value) exceeds a predetermined threshold, this may indicate an adverse bending. In another example, a reference signal may be a proportion of the two strain measurement devices' resistances in an unbent state. The reference signal may be known from a factory calibration or received signal(s) in an unbent state. Then, the proportion of the two strain measurement devices' resistances (deformation value) may be determined from the received signals or be the received signal in order to determine whether the substrate is bent. In particular, if a difference of the proportion of the two strain measurement devices' resistances based on the received signal(s) (deformation value) and the reference signal(s) (reference value) exceeds a predetermined threshold, this may indicate an adverse bending.

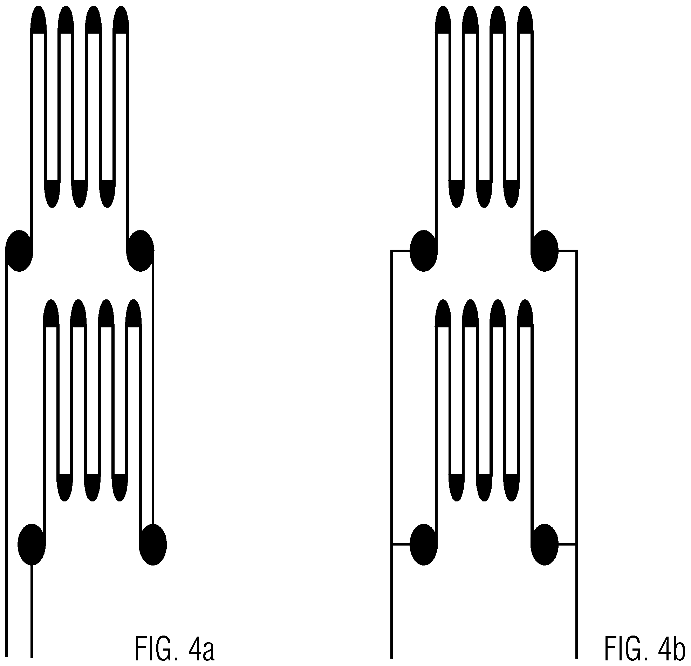

Figs. 4a and 4b show exemplary arrangements for connection of two strain measurement devices, which may be mounted on a flexible insertion piece.

Fig. 4a shows an arrangement of two strain measurement devices connected in series, while Fig. 4b shows an arrangement of two strain measurement devices connected in parallel.

Both of these connection arrangements allow a use of several strain measurement devices with a reduced number of wires being necessary. This may in particular be helpful as the number of contact points for carrying out measurements may therefore be reduced.

However, such a connection in series or in parallel may not allow locating the particular point where the deformation occurs. Hence, the result in deformation information may be the same for different deformations, e.g. a small deformation of each strain measurement device may not be distinguishable from a large deformation of just one of those strain measurement devices. Thus, an arrangement where the strain measurement devices are connected in series and/or in parallel should only be used if the information of the particular point of the deformation along the insertion piece 1 is not of severe importance.

Such an arrangement may e.g. be used for an area in which a deformation would have the same impact if occurring in each part of the area, e.g. along the insertion area. One or more strain measurement devices unconnected to, in particular independent of, the strain measurement device(s) arranged along the insertion area may be used in the connection area, if deformation should also be detected in the connection area in which a different deformation may be expected than in the insertion area.

Thus, there may be more than two strain measurement devices mounted on a flexible insertion piece, also in the same area, e.g. in the insertion area and/or the connection area, and each one may be connected in series, in parallel or unconnected, in particular independent, from the other strain measurement devices mounted on the flexible insertion piece.

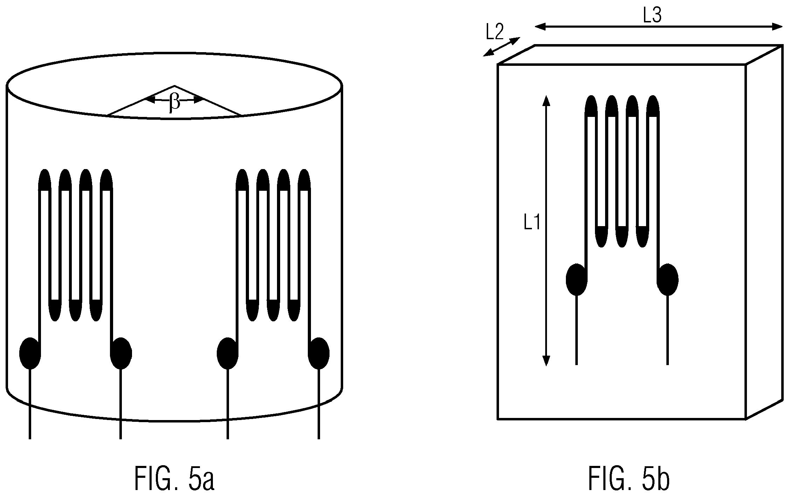

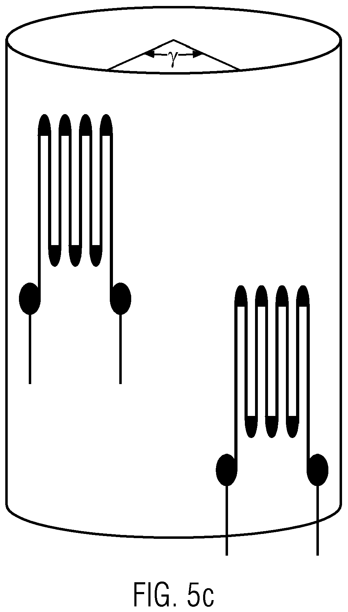

Figs. 5a, 5b and 5c show exemplary arrangements of strain measurement devices on flexible insertion pieces.

In particular, Fig. 5a shows an arrangement of two unconnected, in particular individually wired, strain measurement devices on a flexible insertion piece having a round (circular) cross-section (along a longitudinal axis). In this particular example, the exemplary two strain measurement devices are arranged parallel to each other under an angle ß, the angle being for example measured around the longitudinal axis and between the middle of each strain measurement device. In this particular example, the angle may be about 90°. An angle of about 90° may in particular be an angle of between 45° and 135°, in particular be between 60° and 120°, for example between 75° and 105°. Such an angle of about 90° may have the advantage that it allows detection of deformation information when bending the longitudinal axis of the flexible insertion piece in any direction. In the particular example, the strain measurement devices may not be connected with each other. However, in other embodiments, they may be connected in parallel or in series (or some may be connected in series and/or parallel) in order to reduce the number of wires necessary to send information to and receive information from the strain measurement devices. In further embodiments, there may be more than two strain measurement devices arranged on a flexible insertion piece, e.g. three or more, and they may be arranged under different angles, e.g. at about 90° from each other, or under different angles.

In Fig. 5b, a (flat) flexible insertion piece is shown having mounted thereon an exemplary strain measurement device.

Indicated herein are axis L1 (longitudinal axis), L2 (perpendicular to the longitudinal axis and the plane in which the strain measurement device is arranged) and L3 (in the plane where the strain measurement device is arranged and perpendicular to L1). The particular, flat-shaped flexible insertion piece may comprise a stack of (foil or film) layers along axis L2. It may have a larger length along axis L3 than along axis L2. For example, the length along axis L3 may be more than 2 times, or more than 3 times or more than 10 times the length than the length along axis L2.

While one strain measurement device is shown in Fig. 5b, the stack may have more than one, e.g. 2, 3 or more strain measurement devices. These may be unconnected, in particular individually wired, connected in series or connected in parallel, or some may be unconnected, in particular individually wired, connected in series and/or connected in parallel. For example, a second strain measurement device may be mounted on the opposite side (back) of the flexible insertion piece of Fig. 5b (not visible) arranged parallel (i.e. same height along longitudinal axis L1) and/or at an angle of about 180° around L1.

This may in particular allow achieving more exact deformation information because the arrangement allows detecting an extension of one strain measurement device at the same time as a compression of the other strain measurement device when the insertion piece's longitudinal axis is bent towards the direction of L2 at a height where the strain measurement device is present.

Fig. 5c shows an exemplary arrangement of two strain measurement devices mounted on a flexible insertion piece arranged under an angle γ with regard to each other. The angle may be about 90°, in particular e.g. between 45° and 135°, e.g. between 75° and 105°. The strain measurement devices are arranged at different heights along a longitudinal axis of the flexible insertion piece and are individually wired (unconnected with each other). This arrangement may allow determining the direction and the point of the deformation, in particular its height along the longitudinal axis.

In other examples, there may be more than two strain measurement devices arranged on a flexible insertion piece, e.g. three or more, and they may be arranged under different angles, e.g. at about 90° from each other and/or at different heights from each other. They may be individually wired.

Optionally, the flexible insertion piece of Fig. 5a and/or Fig. 5b and/or Fig. 5c may have one or more further measurement arrangements, e.g. an, optionally electronic, glucose measurement arrangement arranged, e.g. near its tip. Alternatively or additionally, they may have an opening or be shaped as a tube or cannula to allow transcutaneous delivery of a drug, e.g. insulin.

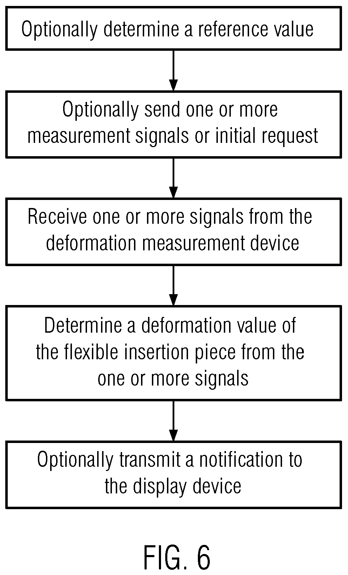

Fig. 6 shows steps which may be comprised in an exemplary method of detecting deformation of at least a part of the insertion piece of an insertion device. A memory of an insertion system may also comprise instructions that, when executed, cause one or more processors to do the corresponding steps.

In particular, the method comprises the step of receiving one or more signals from the deformation measurement device.

These signals may be received in response to one or more measurement signals or an initial request, e.g. from an electronic component (electronics). This electronic component may be comprised in the system and controlled by the memory or be a component outside of the system, e.g. an external device, e.g. cell phone, forwarding the initial request to the memory.

The received signal(s) may allow determining the deformation value of the flexible insertion piece, e.g. by determination of a resistance of one or more strain measurement devices or another deformation measurement device, and comparison thereof to a reference value, e.g. a reference resistance.

A reference value, e.g. a reference resistance for a strain measurement device, may be known from a calibration step carried out before the step of receiving one or more signals from the deformation measurement device. In particular, this reference value may be known from a previous measurement, which may allow a determination whether a change in the deformation, e.g. a bending, has occurred since a previous measurement, e.g. during use, a factory calibration, which may avoid the need for measurement before insertion of the insertion piece or a calibration step determining a reference value before insertion of the insertion device into the skin, which may have the advantage that the calibration is carried out right before use and can thus take into account environmental conditions and/or particularities of the system used.

In particular, for flexible insertion pieces oriented in a bending orientation before insertion corresponding to a bending orientation after insertion, e.g. in the insertion area, the following steps may be caused by the memory: receiving one or more signals from the deformation measurement device before insertion of the flexible insertion piece, determining a reference value therefrom, wherein the reference value may in particular be the one or more signals received from the deformation measurement device before insertion or a value derived therefrom, receiving one or more signals from the deformation measurement device after insertion of the flexible insertion device, determining the deformation value of the flexible insertion piece from the one or more signals, e.g. by using the one or more signals or a value derived therefrom. Then, the determined deformation value may be compared with a reference value. If adverse deformation is noted, optionally, a notification is given to the user, e.g. using the optional monitor. This may in particular be the case if the determined deformation value fulfills a predetermined condition with regard to the reference value.

For flexible insertion pieces oriented in a bending orientation before insertion different from the bending orientation after insertion, e.g. in the connection area, a factory reference value may be used, in particular without the step of receiving one or more signals from the deformation measurement device before insertion of the flexible insertion piece and determining a reference value therefrom. The other steps may, for example, be carried out as described above.

The one or more signals from the deformation measurement device may be measured using electronic equipment (electronics) arranged on the device for transcutaneous insertion or, optionally, outside thereof, e.g. on a patch for attaching the device for transcutaneous insertion to the user's skin, which may be comprised in the insertion system or be external therefrom. For example, a resistance measurement of one or more strain measurement devices may be carried out using a Wheatstone Bridge. The one or more signals received from the deformation measurement device may for example correspond to the resistance. In other embodiments, they may correspond to a value derived from the one or more signals received from the deformation measurement device.

In the following table, the reference signs and the element associated with this number are indicated:

| **Reference number** | **Element** |
|---|---|
| 1 | flexible insertion piece |
| 1a | tip |
| 1b | insertion area |
| 1c | connection area |
| 1d | attachment area |
| 2 | glucose measurement arrangement |
| 3 | strain measurement device |
| 3a | wiring |
| 3b | pads |
| 3c | meander-shaped pattern |
| 4 | skin |
| 5 | patch |
| 5a | opening |
| 6 | housing |
| 7 | cannula |
| 7b | insertion area |
| 8 | protective layer |
| 9 | opening |

## Claims

1. A device for transcutaneous insertion comprising a flexible insertion piece (1) for transcutaneous insertion, the flexible insertion piece (1) comprising a deformation measurement device for detecting one or more deformation signals, wherein the deformation measurement device comprises two strain measurement devices (3).

2. The device of claim 1, wherein the deformation measurement device comprises a strain measurement device (3), in particular for detecting a bending of the insertion piece.

3. The device of claim 2, wherein upon bending and unbending the strain measurement device (3) reverts to its original properties.

4. The device of claims 1-3, wherein the strain measurement device (3) is suitable for continuous measurement and/or an electronic strain measurement device.

5. The device of claims 1-4, wherein the deformation measurement device comprises three or more strain measurement devices (3), and/or wherein optionally the two, three or more strain measurement devices (3) are connected in parallel or wherein optionally the two, three or more strain measurement devices (3) are connected in series.

6. The device of claim 5, wherein two, three or more strain measurement devices (3) are arranged at different angles (β,γ) around a longitudinal axis (L1) of the flexible insertion piece (1).

7. The device of claims 1- 6, wherein an axis of the deformation measurement device is oriented along a longitudinal axis (L1) of the flexible insertion piece (1) and/or wherein the wiring (3a) attached to the deformation measurement device is oriented along a longitudinal axis (L1) of the flexible insertion piece (1).

8. The device of claims 1-7, wherein the deformation measurement device is arranged on the outside of the flexible insertion piece (1) or it is arranged on the inside of the flexible insertion piece off-centered.

9. The device of claims 1-8 wherein the device for transcutaneous insertion is a continuous analyte measurement sensor device comprising a transcutaneous analyte sensor, in particular comprising an electronic analyte measurement arrangement (2).

10. The device of claims 1-8, wherein the flexible insertion piece (1) is a flexible cannula for drug delivery.

11. A transcutaneous insertion system, comprising
a device for transcutaneous insertion according to claims 1-10,
an optional display device, and
a memory, the memory comprising instructions that, when executed, cause one or more processors to:
receive one or more signals from the deformation measurement device;
determine a deformation value of the flexible insertion piece from the one or more signals;
optionally transmit a notification to the display device.

12. A transcutaneous insertion system of claim 11, the memory further comprising instructions that, when executed, cause one or more processors to:
compare the determined deformation value with a reference value,
if the determined deformation value fulfills a predetermined condition with regard to the reference value, conduct at least one of:
a) issue an alarm which is detectable on the device for transcutaneous insertion,
b) issue an alarm which is transmitted to the display device, and
c) deactivate the device for transcutaneous insertion.

13. The transcutaneous insertion system according to claim 11 or 12, wherein the device for transcutaneous insertion is a device for transcutaneous insertion according to claim 9, wherein the optional display device and the memory is comprised in a housing adapted for placement adjacent to skin of a user.

14. A method of detecting deformation of at least a part of the insertion piece of a device for transcutaneous insertion, in particular a device for transcutaneous insertion according to claims 1-10, the method comprising
receiving one or more signals from a deformation measurement device, the deformation measurement device comprising two strain measurement devices (3);
determining a deformation value of the flexible insertion piece from the one or more signals;
optionally transmitting a notification to a display device.

15. The method of detecting deformation according to claim 14, the method comprising
comparing the determined deformation value with a reference value,
if the determined deformation value fulfills a predetermined condition with regard to the reference value, conducting at least one of:
a) issue an alarm which is detectable on the device for transcutaneous insertion,
b) issue an alarm which is transmitted to the display device, and
c) deactivate the device for transcutaneous insertion.

## Patentansprüche

1. Vorrichtung zur transkutanen Einführung, umfassend ein flexibles Einführstück (1) zur transkutanen Einführung, wobei das flexible Einführstück (1) eine Verformungsmessvorrichtung zum Erfassen eines Verformungssignals oder mehrerer Verformungssignale umfasst, wobei die Verformungsmessvorrichtung zwei Dehnungsmessvorrichtungen (3) umfasst.

2. Vorrichtung nach Anspruch 1, wobei die Verformungsmessvorrichtung eine Dehnungsmessvorrichtung (3), insbesondere zum Erfassen eines Biegens des Einführstücks, umfasst.

3. Vorrichtung nach Anspruch 2, wobei die Dehnungsmessvorrichtung (3) nach Biegen und Geradebiegen zu ihren ursprünglichen Eigenschaften zurückkehrt.

4. Vorrichtung nach den Ansprüchen 1-3, wobei die Dehnungsmessvorrichtung (3) für kontinuierliche Messung geeignet und/oder eine elektronische Dehnungsmessvorrichtung ist.

5. Vorrichtung nach den Ansprüchen 1-4, wobei die Verformungsmessvorrichtung drei oder mehr Dehnungsmessvorrichtungen (3) umfasst und/oder wobei gegebenenfalls die zwei, drei oder mehr Dehnungsmessvorrichtungen (3) parallel verbunden sind oder wobei gegebenenfalls die zwei, drei oder mehr Dehnungsmessvorrichtungen (3) in Reihe verbunden sind.

6. Vorrichtung nach Anspruch 5, wobei zwei, drei oder mehr Dehnungsmessvorrichtungen (3) in verschiedenen Winkeln (β, γ) um eine Längsachse (L1) des flexiblen Einführstücks (1) angeordnet sind.

7. Vorrichtung nach den Ansprüchen 1-6, wobei eine Achse der Verformungsmessvorrichtung entlang einer Längsachse (L1) des flexiblen Einführstücks (1) ausgerichtet ist und/oder wobei die Verdrahtung (3a), die an der Verformungsmessvorrichtung befestigt ist, entlang einer Längsachse (L1) des flexiblen Einführstücks (1) ausgerichtet ist.

8. Vorrichtung nach den Ansprüchen 1-7, wobei die Verformungsmessvorrichtung an der Außenseite des flexiblen Einführstücks (1) angeordnet ist oder an der Innenseite des flexiblen Einführstücks außermittig angeordnet ist.

9. Vorrichtung nach den Ansprüchen 1-8, wobei die Vorrichtung zur transkutanen Einführung eine Sensorvorrichtung für kontinuierliche Analytmessung ist, die einen transkutanen Analytsensor umfasst, insbesondere eine elektronische Analytmessanordung (2) umfasst.

10. Vorrichtung nach den Ansprüchen 1-8, wobei das flexible Einführstück (1) eine flexible Kanüle für Arzneimittelgabe ist.

11. Transkutanes Einführsystem, umfassend
eine Vorrichtung zur transkutanen Einführung nach den Ansprüchen 1-10,
eine optionale Anzeigevorrichtung und
einen Speicher, wobei der Speicher Anweisungen umfasst, die beim Ausführen einen oder mehrere Prozessor(en) zu Folgendem veranlassen:
Empfangen eines Signals oder mehrerer Signale von der Verformungsmessvorrichtung;
Bestimmen eines Verformungswertes des flexiblen Einführstücks aus dem einen oder den mehreren Signal(en);
gegebenenfalls Senden einer Mitteilung an die Anzeigevorrichtung.

12. Transkutanes Einführsystem nach Anspruch 11, wobei der Speicher ferner Anweisungen umfasst, die beim Ausführen einen oder mehrere Prozessor(en) zu Folgendem veranlassen:
Vergleichen des bestimmten Verformungswertes mit einem Referenzwert,
wenn der bestimmte Verformungswert eine vorbestimmte Bedingung in Bezug auf den Referenzwert erfüllt, Ausführen von mindestens einem von Folgendem:
a) Ausgeben eines Alarms, der an der Vorrichtung zur transkutanen Einführung erfasst werden kann,
b) Ausgeben eines Alarms, der an die Anzeigevorrichtung gesendet wird, und
c) Deaktivieren der Vorrichtung zur transkutanen Einführung.

13. Transkutanes Einführsystem nach Anspruch 11 oder 12, wobei die Vorrichtung zur transkutanen Einführung eine Vorrichtung zur transkutanen Einführung nach Anspruch 9 ist, wobei die optionale Anzeigevorrichtung und der Speicher in einem Gehäuse umfasst sind, das dafür ausgelegt ist, anliegend an der Haut eines Benutzers platziert zu werden.

14. Verfahren zum Erfassen von Verformung mindestens eines Teils des Einführstücks einer Vorrichtung zur transkutanen Einführung, insbesondere einer Vorrichtung zur transkutanen Einführung nach den Ansprüchen 1-10, wobei das Verfahren Folgendes umfasst
Empfangen eines Signals oder mehrerer Signale von einer Verformungsmessvorrichtung, wobei die Verformungsmessvorrichtung zwei Dehnungsmessvorrichtungen (3) umfasst; Bestimmen eines Verformungswertes des flexiblen Einführstücks aus dem einen oder den mehreren Signal(en);
gegebenenfalls Senden einer Mitteilung an eine Anzeigevorrichtung.

15. Verfahren zum Erfassen von Verformung nach Anspruch 14, wobei das Verfahren Folgendes umfasst
Vergleichen des bestimmten Verformungswertes mit einem Referenzwert,
wenn der bestimmte Verformungswert eine vorbestimmte Bedingung in Bezug auf den Referenzwert erfüllt, Ausführen von mindestens einem von Folgendem:
a) Ausgeben eines Alarms, der an der Vorrichtung zur transkutanen Einführung erfasst werden kann,
b) Ausgeben eines Alarms, der an die Anzeigevorrichtung gesendet wird, und
c) Deaktivieren der Vorrichtung zur transkutanen Einführung.

## Revendications

1. Dispositif pour insertion transcutanée comprenant une pièce d'insertion souple (1) pour insertion transcutanée, la pièce d'insertion souple (1) comprenant un dispositif de mesure de déformation pour détecter un ou plusieurs signaux de déformation, dans lequel le dispositif de mesure de déformation comprend deux dispositifs de mesure de contrainte (3).

2. Dispositif selon la revendication 1, dans lequel le dispositif de mesure de déformation comprend un dispositif de mesure de contrainte (3), en particulier pour détecter une flexion de la pièce d'insertion.

3. Dispositif selon la revendication 2, dans lequel lors de la flexion et de la détente du dispositif de mesure de contrainte (3) revient à ses propriétés d'origine.

4. Dispositif selon les revendications 1 à 3, dans lequel le dispositif de mesure de contrainte (3) est approprié pour la mesure en continu et/ou un dispositif de mesure de contrainte électronique.

5. Dispositif selon les revendications 1 à 4, dans lequel le dispositif de mesure de déformation comprend trois dispositifs de mesure de contrainte (3) ou plus, et/ou dans lequel éventuellement les deux, trois ou plus dispositifs de mesures de contrainte (3) sont connectées en parallèle ou dans lequel éventuellement les deux, trois ou plus dispositifs de mesure de contrainte (3) sont connectés en série.

6. Dispositif selon la revendication 5, dans lequel deux, trois ou plus dispositifs de mesure de contrainte (3) sont agencés à différents angles (β,γ) autour d'un axe longitudinal (L1) de la pièce d'insertion souple (1).

7. Dispositif selon les revendications 1 à 6, dans lequel un axe du dispositif de mesure de déformation est orienté le long d'un axe longitudinal (L1) de la pièce d'insertion souple (1) et/ou dans lequel le câblage (3a) attaché au dispositif de mesure de déformation est orienté le long d'un axe longitudinal (L1) de la pièce d'insertion souple (1).

8. Dispositif selon les revendications 1 à 7, dans lequel le dispositif de mesure de déformation est agencé sur l'extérieur de la pièce d'insertion souple (1) ou il est agencé sur l'intérieur de la pièce d'insertion souple de manière décentrée.

9. Dispositif selon les revendications 1 à 8 dans lequel le dispositif pour insertion transcutanée est un dispositif de capteur de mesure d'analyte en continu comprenant un capteur d'analyte transcutané, en particulier comprenant un agencement de mesure d'analyte électronique (2).

10. Dispositif selon les revendications 1 à 8, dans lequel la pièce d'insertion souple (1) est une canule souple pour administration de médicament.

11. Système d'insertion transcutanée, comprenant
un dispositif pour insertion transcutanée selon les revendications 1 à 10,
un dispositif d'affichage facultatif, et
une mémoire, la mémoire comprenant des instructions qui, lorsqu'elles sont exécutées, amènent un ou plusieurs processeurs à :
recevoir un ou plusieurs signaux à partir du dispositif de mesure de déformation ;
déterminer une valeur de déformation de la pièce d'insertion souple à partir du ou des signaux ;
éventuellement transmettre une notification au dispositif d'affichage.

12. Système d'insertion transcutanée selon la revendication 11, la mémoire comprenant en outre des instructions qui, lorsqu'elles sont exécutées, amènent un ou plusieurs processeurs à :
comparer la valeur de déformation déterminée à une valeur de référence,
si la valeur de déformation déterminée remplit une condition prédéterminée par rapport à la valeur de référence, réaliser au moins un parmi :
a) émettre une alarme qui est détectable sur le dispositif pour insertion transcutanée,
b) émettre une alarme qui est transmise au dispositif d'affichage, et
c) désactiver le dispositif pour insertion transcutanée.

13. Système d'insertion transcutanée selon la revendication 11 ou 12, dans lequel le dispositif pour insertion transcutanée est un dispositif pour insertion transcutanée selon la revendication 9, dans lequel le dispositif d'affichage facultatif et la mémoire sont compris dans un boîtier adapté pour un placement adjacent à la peau d'un utilisateur.

14. Procédé de détection d'une déformation d'au moins une partie de la pièce d'insertion d'un dispositif pour insertion transcutanée, en particulier un dispositif pour insertion transcutanée selon les revendications 1 à 10, le procédé comprenant
la réception d'un ou de plusieurs signaux à partir d'un dispositif de mesure de déformation, le dispositif de mesure de déformation comprenant deux dispositifs de mesure de contrainte (3) ;
la détermination d'une valeur de déformation de la pièce d'insertion souple à partir du ou des signaux ;
éventuellement la transmission d'une notification à un dispositif d'affichage.

15. Procédé de détection d'une déformation selon la revendication 14, le procédé comprenant la comparaison de la valeur de déformation déterminée à une valeur de référence,
si la valeur de déformation déterminée remplit une condition prédéterminée par rapport à la valeur de référence, réaliser au moins un parmi :
a) émettre une alarme qui est détectable sur le dispositif pour insertion transcutanée,
b) émettre une alarme qui est transmise au dispositif d'affichage, et
c) désactiver le dispositif pour insertion transcutanée.
